# EUROPEAN PATENT APPLICATION

(11) **EP 4 356 834 A1**
(43) Date of publication of application: **24.04.2024**
(21) Application number: 22849165.0
(22) Date of filing: 01.07.2022
(51) Int. Cl.: A61B 5/16

(54) **SLEEP/WAKING DETERMINATION SYSTEM, SLEEP/WAKING DETERMINATION METHOD, AND PROGRAM**

(30) Priority: 27.07.2021 JP 2021122223
(71) Applicant: Accelstars, Inc., Kurume-shi, Fukuoka 839-0864 (JP)
(72) Inventor: SHI, Shoi, Kurume-shi, Fukuoka 839-0864 (JP); KATORI, Machiko, Kurume-shi, Fukuoka 839-0864 (JP); YAMADA, Rikuhiro, Kurume-shi, Fukuoka 839-0864 (JP); UEDA, Hiroki, Kurume-shi, Fukuoka 839-0864 (JP)
(74) Representative: Hasegawa, Kan
(86) International application number: PCT/JP2022/026470
(87) International publication number: WO 2023/008099

(57) **Abstract**

[Problem] To provide a sleep/waking determination system and the like that determine sleep and waking with substantially high accuracy using few worn instruments. [Solution] One aspect of the present invention provides a sleep/waking determination system. The sleep/waking determination system comprises at least one processor that can execute a program to perform the following steps. An acquisition step for acquiring a signal that indicates the acceleration of at least a portion of the body of a user. A band limiting step for limiting a frequency component of the signal that indicates the acceleration to a specific frequency component. A transformation step for performing a Fourier transformation on a signal that consists of the specific frequency component to generate determination data. A determination step for determining the sleep and waking of the user on the basis of the determination data and preset reference data.

## Description

### BACKGROUND

The present invention relates to a sleep and arousal determination system, a sleep and arousal determination method, and a program.

### RELATED ART

Ensuring healthy sleep is necessary to maintain good health, and sleep disorders such as insomnia, sleep-disordered breathing, and hypersomnia are known to cause health problems. To understand sleeping state of a person, it is necessary to test an actual condition of how the person actually sleeps, either overnight or over a period of several days.

Polysomnography (PSG), as proposed in Patent Document 1, has been developed as a method for testing sleeping state of a person. PSG measures basic data such as electroencephalogram (EEG), electrocardiogram (ECG), electromyogram (EMG), and respiratory status by attaching numerous electrodes and sensors to a body of a person being tested, and connecting each electrode and sensor to a dedicated measurement device.

### PRIOR ART DOCUMENTS

### Patent document

[Patent Document 1] JP 2013-99507 A

### SUMMARY

### Problems to be Solved by Invention

In sleep test using PSG, such as in Patent Document 1, many measurement devices are used, and a testing location is limited to hospital or laboratory. This makes it difficult for many people to casually undergo long-term testing over several days. In addition, wearing many electrodes and sensors on a body in an environment different from home also causes stress and makes it difficult to sleep. Therefore, it is difficult to correctly test the usual sleep state.

In view of the above circumstances, the present invention provides a sleep and arousal determination system or the like that determines sleep and arousal with sufficiently high accuracy using a small number of wearing device.

### Means for Solving Problems

According to an aspect of the present invention, a sleep and arousal determination system is provided. The sleep and arousal determination system comprises at least one processor configured to execute a program to cause each of following steps to be performed: an acquisition step of acquiring a signal indicating acceleration of at least a part of body of a user; a bandwidth limitation step of limiting a frequency component included in the signal indicating the acceleration to a specific frequency component; a transform step of Fourier transforming a signal configured of the specific frequency component to generate data for determination; and a determination step of determining sleep and arousal of the user based on the data for determination and preset reference information.

According to such an aspect, it becomes possible to determine sleep and arousal with sufficiently high accuracy using a small number of wearing device.

### BRIEF DESCRIPTION OF DRAWINGS

FIG. 1 is a diagram showing an overview of a configuration of a sleep and arousal determination system 1 according to the present embodiment.
FIG. 2 is a block diagram showing a hardware configuration of the sleep and arousal determination system 1 shown in FIG. 1.
FIG. 3 is a photograph showing an example of a wearable device 2.
FIG. 4 is a functional block diagram of a controller 33 in an information processing apparatus 3.
FIG. 5 is an activity diagram showing information processing flow of the sleep and arousal determination system 1.
FIG. 6 is a conceptual diagram for illustrating epoch E.
FIG. 7 is a conceptual diagram for illustrating epoch E.
FIG. 8 is a diagram in which a graph shows L2 norm of acceleration per epoch (FIG. 8A) and a hypnogram shows actual sleep (FIG. 8B).
FIG. 9 is a graph showing accuracy of a learned model IF1 by sampling frequency.
FIG. 10 is a graph showing accuracy of sleep and arousal determination by bit rate in analog-to-digital conversion.
FIG. 11 is a schematic diagram showing standardization processing by a correction unit 334.

### DETAILED DESCRIPTION

Hereinafter, embodiment of the present invention will be described with reference to the drawings. Various features described in the embodiment below can be combined with each other.

A program for realizing a software in the present embodiment may be provided as a non-transitory computer readable medium that can be read by a computer or may be provided for download from an external server or may be provided so that the program can be activated on an external computer to realize functions thereof on a client terminal (so-called cloud computing).

In the present embodiment, the "unit" may include, for instance, a combination of hardware resources implemented by a circuit in a broad sense and information processing of software that can be concretely realized by these hardware resources. Further, various information is performed in the present embodiment, and the information can be represented by, for instance, physical values of signal values representing voltage and current, high and low signal values as a set of binary bits consisting of 0 or 1, or quantum superposition (so-called qubits), and communication/calculation can be executed on a circuit in a broad sense.

Further, the circuit in a broad sense is a circuit realized by combining at least an appropriate number of a circuit, a circuitry, a processor, a memory, or the like. In other words, it is a circuit includes application specific integrated circuit (ASIC), programmable logic device (e.g., simple programmable logic device (SPLD), complex programmable logic device (CPLD), field programmable gate array (FPGA)), or the like.

### 1. Hardware configuration

In this section, an overall configuration of a sleep and arousal determination system 1 will be described.

### 1.1 Sleep and arousal determination system 1

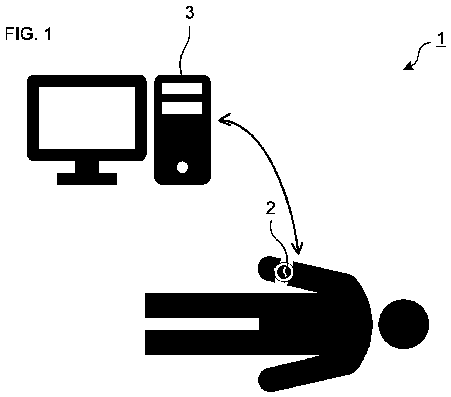

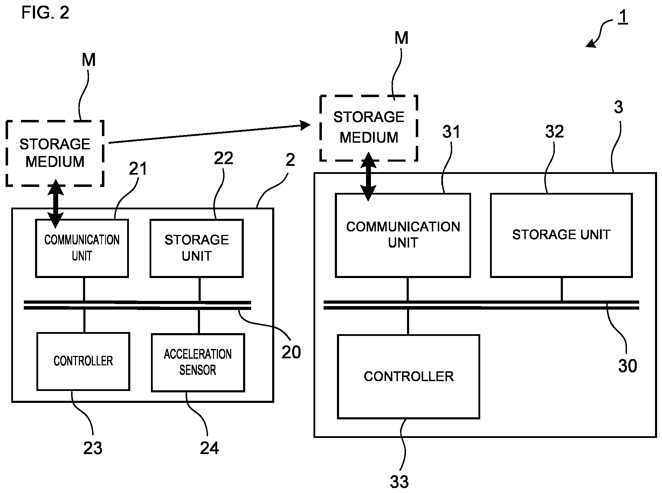

FIG. 1 shows an overview of a configuration of the sleep and arousal determination system 1 according to the present embodiment. The sleep and arousal determination system 1 comprises a wearable device 2 and an information processing apparatus 3, and these components may exchange information through electrical communication means. FIG. 2 is a block diagram showing a hardware configuration of the sleep and arousal determination system 1 shown in FIG. 1.

### 1.2 Wearable device 2

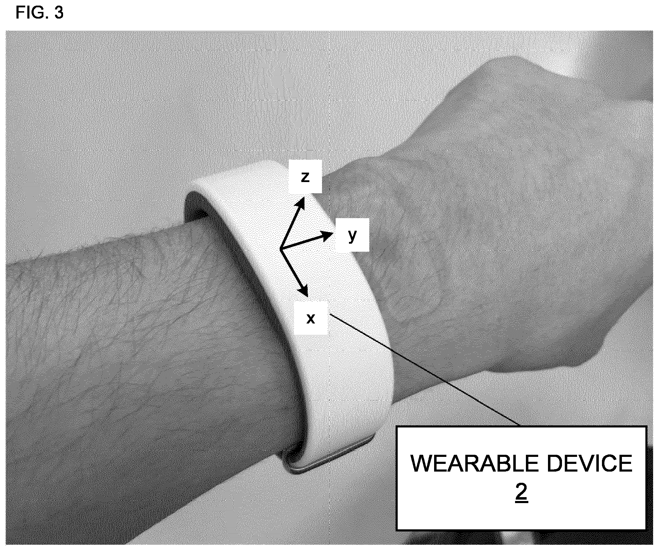

FIG. 3 is a photograph showing an example of a wearable device 2. As shown in FIG. 3, the wearable device 2 is a small device that can be worn, for example, on an arm of a user. Further, as shown in FIG. 2, the wearable device 2 includes a communication unit 21, a storage unit 22, a controller 23, and an acceleration sensor 24, and these components are electrically connected inside the wearable device 2 via communication bus 20. Hereinafter, each component will be further described.

The communication unit 21 is preferably designed to have wired type communication means such as USB, IEEE 1394, Thunderbolt (registered trademark), wired LAN network communication, etc., as well as wireless LAN network communication, mobile communication such as 3G/LTE/5G, Bluetooth (registered trademark) communication, etc. as necessary. In particular, in the present embodiment, it is preferable that the communication unit 21 be configured to write information including a time-series three-dimensional acceleration vector v (x, y, z) measured by the acceleration sensor 24 (described below) to an external storage medium M. Type and manner of the storage medium M is not particularly limited, for instance, flash memory, card-type memory, optical disk, etc. may be employed as appropriate.

The storage unit 22 is configured to store various information as defined by the above description. It can be implemented as a storage device, such as a solid state drive (SSD), or as a memory such as a random access memory (RAM) that stores temporarily necessary information (argument, sequence, etc.) for program operation. Moreover, a combination thereof may be applied. In particular, information including a time-series three-dimensional acceleration vector v (x, y, z) measured by the acceleration sensor 24 described later can be stored. Note that it may be implemented in such a manner that information is stored directly on the storage medium M without going through the storage unit 22.

The controller 23 performs processing and control of overall operation related to the wearable device 2. The controller 23 is, for example, an unshown CPU (Central Processing Unit). The controller 23 is configured to realize various functions related to the wearable device 2 by reading a predetermined program stored in the storage unit 22. In other words, information processing by software stored in the storage unit 22 is specifically realized by the controller 23, which is an example of hardware, and can be executed as each function execution unit included in the controller 23. The controller 33 is not limited to being a single unit, but may be implemented in such a manner that there are two or more controllers 23 for each function. Moreover, a combination thereof may be applied.

The acceleration sensor 24 is configured to measure acceleration of a part of body of a user, e.g., arm, as three-dimensional vector information. That is, information including a time-series three-dimensional acceleration vector v (x, y, z) can be acquired from the user.

### 1.3 Information processing apparatus 3

As shown in FIG. 2, an information processing apparatus 3 includes a communication unit 31, a storage unit 32, and a controller 33, and these components are electrically connected via communication bus 30 within the information processing apparatus 3. Hereinafter, each component will be further described.

The communication unit 31 is preferably designed to have wired type communication means such as USB, IEEE 1394, Thunderbolt (registered trademark), wired LAN network communication, etc., as well as wireless LAN network communication, mobile communication such as 3G/LTE/5G, Bluetooth (registered trademark) communication, etc. as necessary. In particular, in the present embodiment, it is preferable to implement the communication unit 31 as a storage medium reading unit that can read information stored in an external storage medium M. The storage medium M stores information including a time-series three-dimensional acceleration vector v (x, y, z) obtained from the user by the wearable device 2. Thereby, the communication unit 31, which is the storage medium reading unit, is configured to read the three-dimensional acceleration vector v (x, y, z) stored in the storage medium M.

The storage unit 32 is configured to store various information as defined by the above description. This can be implemented as a storage device, such as a solid state drive (SSD), or as a memory such as a random access memory (RAM) that stores temporarily necessary information (argument, sequence, etc.) for program operation. Moreover, a combination thereof may be applied. In particular, the storage unit 32 is configured to store various programs, etc. with respect to the information processing apparatus 3 to be executed by the controller 33.

Furthermore, the storage unit 32 is configured to store a learned model that has learned correlation between a feature quantity f(N) of a desired epoch, a feature quantity f(N±δ) of a recent epoch, and sleep and arousal of the user. As such a machine learning algorithm, it is preferable to adopt a conventional algorithm as appropriate. Examples include Logistic Regression, Random Forest, XGBoost (Extreme Gradient Boosting), Multilayer Perceptron (MLP), or the like. Further, each time the information processing apparatus 3 is used, machine learning can be further performed using this as the teaching data to update the learned model.

The controller 33 (an example of a processor) performs processing and control of overall operation related to the information processing apparatus 3. The controller 33 is, for instance, an unshown CPU (Central Processing Unit). The controller 33 reads a predetermined program stored in the storage unit 32 to realize various functions related to the information processing apparatus 3. In other words, information processing by software (stored in the storage unit 32) is specifically realized by hardware (the controller 33), and can be executed as each function execution unit described below. In FIG. 2, the controller 33 is described as a single controller, but it is actually not limited thereto, and may be implemented with two or more controllers for each function. Further, a combination thereof may be applied.

### 2. Functional configuration

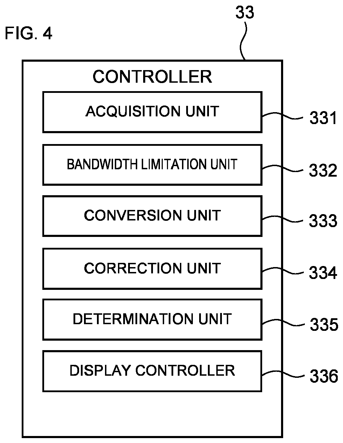

In this section, a functional configuration according to the present embodiment will be described. As mentioned above, information processing by software stored in the storage unit 32 is specifically realized by the controller 33, which is an example of hardware, and can be executed as each function execution unit included in the controller 33. FIG. 4 is a functional block diagram of the controller 33 in the information processing apparatus 3. That is, the controller 33 comprises following units.

An acquisition unit 331 is configured to acquire various types of information from external sources, such as through a network or a storage medium M. For example, the acquisition unit 331 may acquire a signal indicating acceleration of at least a part of body of a user. This will be described in further detail later.

A bandwidth limitation unit 332 is configured to execute bandwidth limitation processing on the signal acquired by the acquisition unit 331. For instance, the bandwidth limitation unit 332 may limit a frequency component included in the signal indicating acceleration to a specific frequency component C. This will be described in further detail later.

A conversion unit 333 is configured to execute Fourier transform processing on a signal configured of the specific frequency component C limited by the bandwidth limitation unit 332. For example, a Fourier transform may be executed on the signal configured of the specific frequency component C to generate data for determination. This will be described in further detail later.

A correction unit 334 is configured to execute correction processing. For example, the correction unit 334 may standardize and correct distribution of frequency component of the data for determination generated by the conversion unit 333. This will be described in further detail later.

A determination unit 335 is configured to determine sleep and arousal of a user. For instance, the determination unit 335 may determine sleep and arousal of a user based on the specific frequency component C and preset reference information IF. This will be described in further detail later.

A display controller 336 is configured to generate various types of display information and control display content visible to the user. The display information may be information itself such as screen, image, icon, text, etc. generated in a form that is visible to the user, or may be rendering information for displaying screen, image, icon, text, etc.

### 3. Information processing method

In this section, an information processing method of the sleep and arousal determination system 1 will be described.

### (Outline of flow)

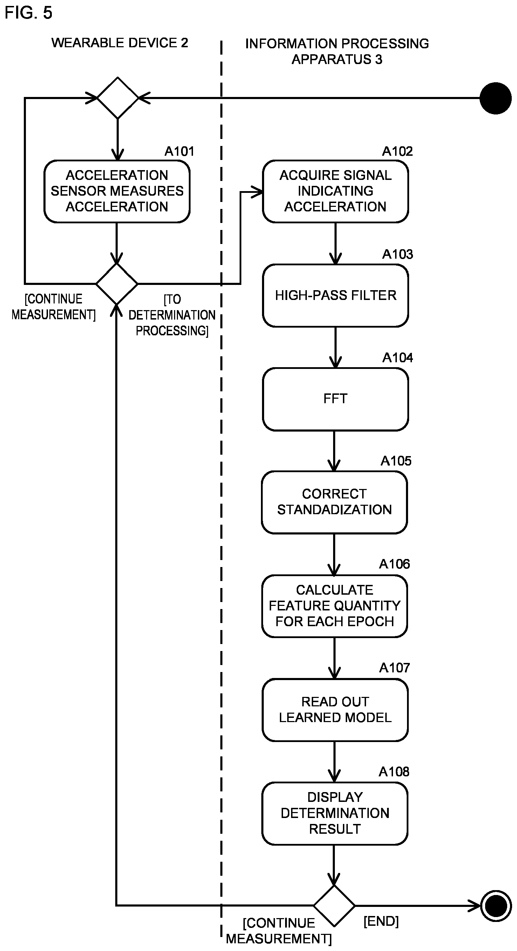

FIG. 5 is an activity diagram showing information processing flow of the sleep and arousal determination system 1. The following outlines information processing flow along each activity in FIG. 5. A user mentioned here is assumed to be a person who wishes to have sleep and arousal determined using service provided by the sleep and arousal determination system 1. Note that sleep and arousal determination here may include determining whether the user is in a sleep state or in an arousal state. The user wears the wearable device 2 on, for example, his/her arm.

First, the user is lying down to rest with the wearable device 2 attached to at least a part of his/her body, e.g., one arm. During this time, the acceleration sensor 24 in the wearable device 2 successively detects and measures acceleration of one arm of the user (Activity A101). Log data of a signal indicating the measured acceleration is sequentially stored in the storage medium M inserted in the wearable device 2.

Subsequently, after sufficient log data of the signal indicating acceleration has been acquired, the signal indicating acceleration stored in the storage medium M is transferred to the information processing apparatus 3 by replacing the storage medium M from the wearable device 2 to the information processing apparatus 3. In other words, the acquisition unit 331 acquires a signal indicating acceleration of at least a part of body of the user (Activity A102). The signal indicating the acquired acceleration may be read out to a temporary storage area of the storage unit 32.

Then, the controller 33 is configured to read a predetermined program stored in the storage unit 32 to limit the frequency component included in the signal indicating the acceleration to the specific frequency component C. That is, the bandwidth limitation unit 332 limits the frequency component included in the signal indicating acceleration to the specific frequency component C. In particular, the bandwidth limitation unit 332 preferably limits the frequency component to the specific frequency component C using a high-pass filter HF (Activity A103). According to such a manner, the specific frequency component C can be restricted to a high frequency component equal to or higher than a cutoff frequency, and sleep and arousal of the user can be determined more robustly.

Then, the controller 33 is configured to read a predetermined program stored in the storage unit 32 to perform Fourier transform on the signal configured of the specific frequency component C. Such Fourier transform is preferably performed using FFT (Fast Fourier Transformation) algorithm. In other words, the conversion unit 333 performs Fourier transform on the signal configured of the specific frequency component C to generate data for determination (Activity A104).

Subsequently, the controller 33 is configured to read a predetermined program stored in the storage unit 32, thereby standardizing frequency component of the generated data for determination. In other words, the correction unit 334 standardizes and corrects the frequency component (distribution of the specific frequency component C) of the data for determination (Activity A105).

The standardized data for determination is then handled as epoch E, which is defined in unit of a predetermined time t. Specifically, the controller 33 reads out a predetermined program stored in the storage unit 32, and the feature quantity f(N) for each epoch E is calculated (Activity A106). N here is a serial number of epoch E, which will be described in further detail later.

Thereafter, the controller 33 is configured to read the reference information IF stored in the storage unit 32 (Activity A 107), and by providing the reference information IF with the feature quantity f of the desired epoch E and the feature quantity f of the epoch E defined around the desired epoch E, a determination result with respect to sleep and arousal of the user is displayed. Particularly preferably, the reference information IF is a learned model IF1 in which correlation between the specific frequency component C and the sleep and arousal has been learned. According to such a manner, sleep and arousal of a user can be determined with high accuracy using machine learning.

In other words, the determination unit 335 is configured to determine sleep and arousal of the user based on the data for determination including the specific frequency component C and the preset reference information IF. Preferably, the determination unit 335 is configured to specify the feature quantity f for each epoch E defined by the predetermined time t based on the specific frequency component C. Further, the determination unit 335 is configured to determine sleep and arousal of the user based on, among the epoch E, the feature quantity f of the desired epoch E, the feature quantity f of the recent epoch E past the desired epoch E in the time series, and the learned model IF1 that is an example of the reference information IF. Then, the display controller 336 is configured to control in such a manner that such determination result can be presented to the user (Activity A108). According to such a manner, sleep and arousal of a user can be determined with higher accuracy.

In summary, the sleep and arousal determination method comprises each step in the sleep and arousal determination system 1. In an acquisition step, a signal indicating the acceleration of at least a part of body of the user is acquired. In a bandwidth limitation step, the frequency component included in the signal indicating acceleration is limited to the specific frequency component C. In a transform step, the signal configuring of the specific frequency component C is Fourier transformed to generate data for determination. In a determination step, sleep and arousal of the user is determined based on the data for determination and the preset reference information.

According to such an approach, it is possible to determine sleep and arousal with a sufficiently high accuracy using a small number of wearing device. In particular, by executing bandwidth limitation first and then Fourier transform, it is possible to make the feature quantity f salient in the data for determination, which in turn contributes to the sleep and arousal determination. In addition, since it does not depend on the sampling frequency at which the apparatus acquires the signal, the sleep and arousal of the user can be determined more robustly.

### (Detail of information processing)

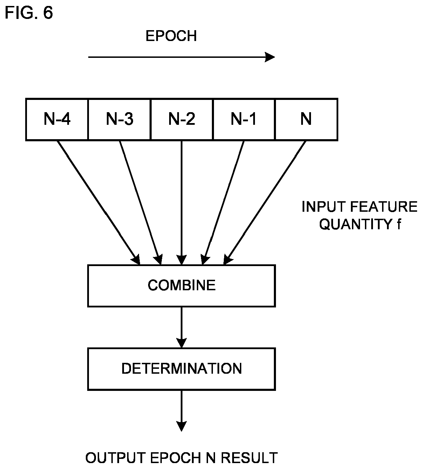

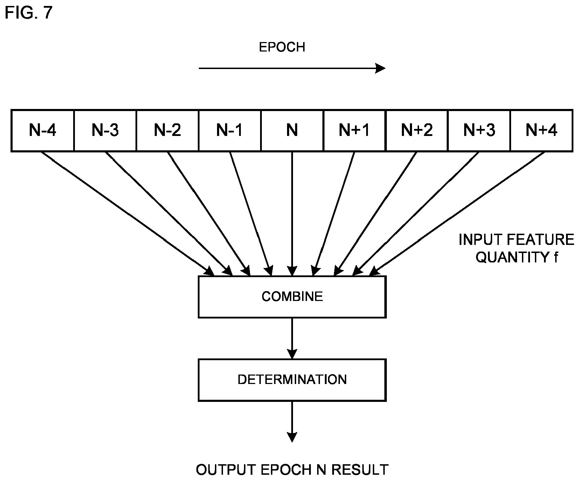

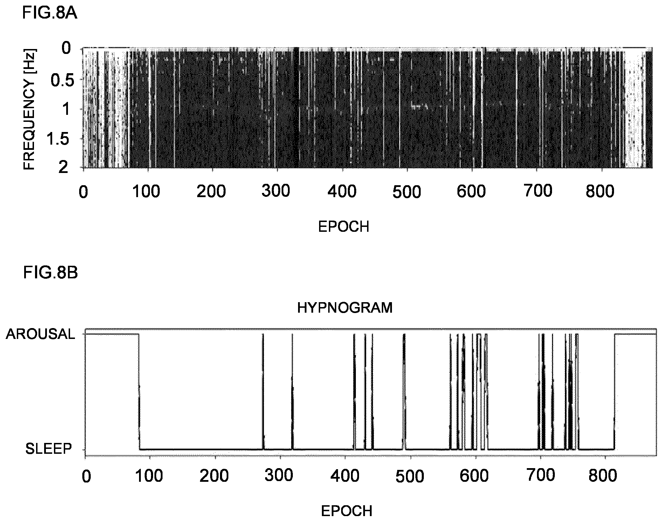

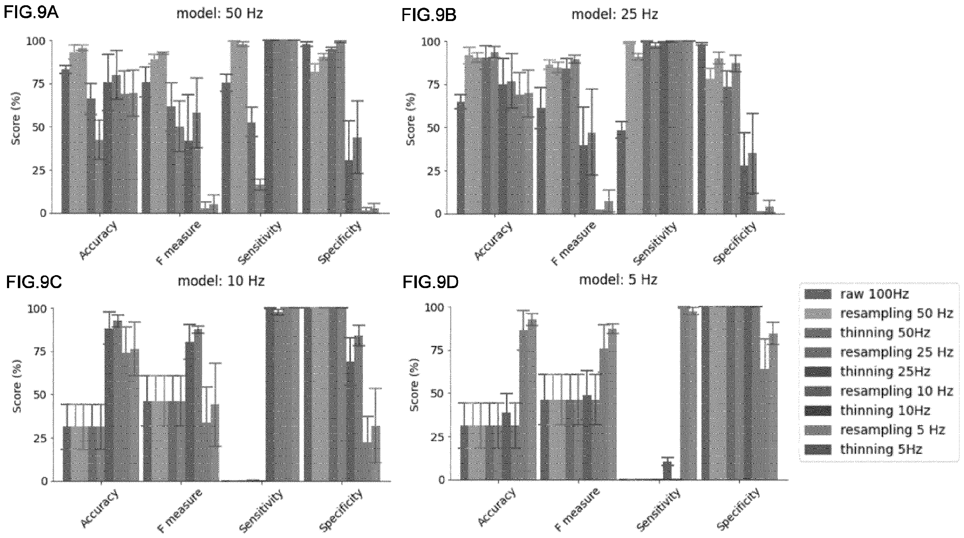

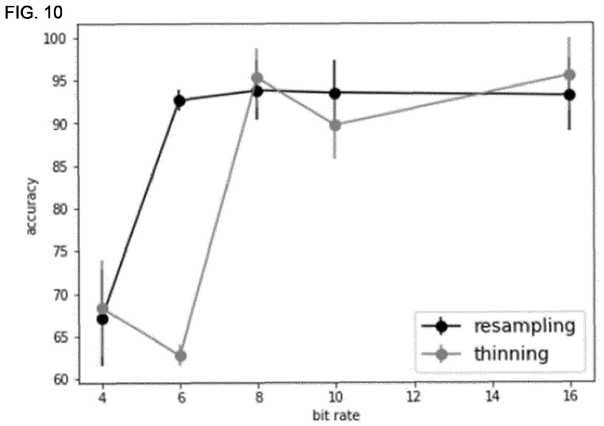

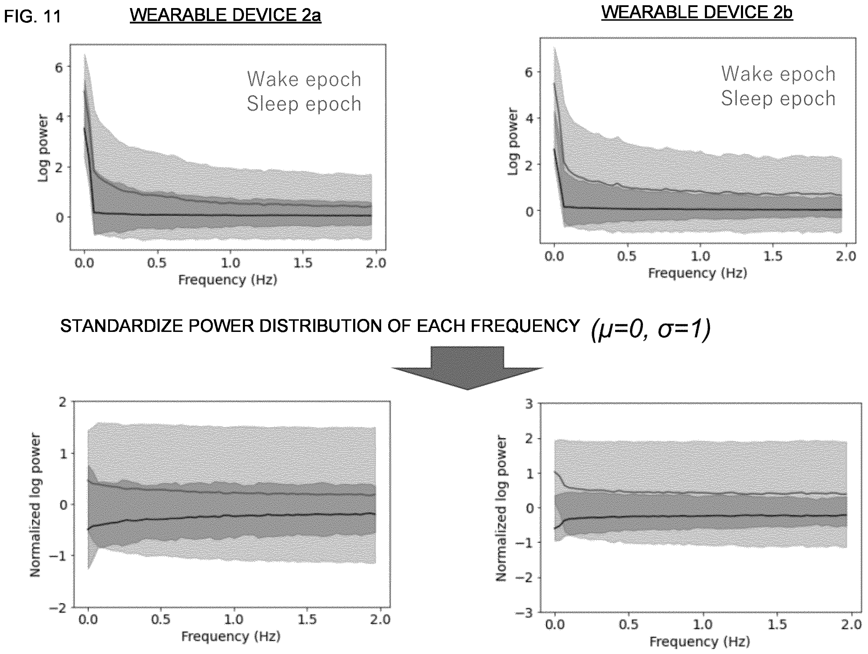

Here, detail of the information processing outlined in FIG. 5 will be illustrated. FIGS. 6 and 7 are conceptual diagrams for illustrating epoch E. FIG. 8 is a diagram in which a graph shows L2 norm of acceleration per epoch (FIG. 8A) and a hypnogram shows actual sleep (FIG. 8B). FIG. 9 is a graph showing accuracy of the learned model IF1 by sampling frequency. FIG. 10 is a graph showing accuracy of the sleep and arousal determination by bit rate in analog-to-digital conversion. FIG. 11 is a schematic diagram showing standardization processing by the correction unit 334.

As shown in FIG. 6, epoch E is signal log data of the acceleration defined by the predetermined time t. This predetermined time t is, for instance, from 5 to 600 seconds, preferably from 10 to 120 seconds, more preferably from 20 to 60 seconds, specifically, for example, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 seconds, and may be in a range between any two of the numerical values exemplified here.

In the present embodiment, the signal indicating acceleration is handled as two or more epochs E. Nth epoch E in the time series is referred to as desired epoch E, and the epoch slightly past the desired epoch E, for example, N-1 to N-4 epochs, are referred to as recent epoch E. In the present embodiment, sleep and arousal of the user is determined based on the aforementioned learned model stored in the storage unit 32, using the feature quantity f(N) of the desired epoch E and the feature quantity f(N-δ) of the recent epoch E as input.

The value of δ may specifically be, for example, 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, and may be in a range between any two of the numerical values exemplified here. Of course, the number of epochs is only an example, and is not limited thereto. Further, instead of the feature quantity f(N-δ), the feature quantity f(N±δ) may be applied as shown in FIG. 7. In such a case, for example, N-4 to N+4th epochs E may be referred to as peripheral epoch E.

Further, the feature quantity f of the epoch E is not particularly limited. For example, a feature quantity f(N) may be extracted from the L2 norm of a high-pass filter HF, FFT and standardization processing applied to the three-dimensional acceleration vector v (x, y, z), etc. representing acceleration, and used to determine sleep and arousal. Specifically, for instance, the feature quantity f(N) may be a histogram generated by dividing a scalar value such as the L2 norm or its logarithm into classes with two or more threshold values, or a power spectrum based on a product value of multiplying a scalar value by a window function. For example, FIG. 8A shows a logarithmic power spectrum of time differences in acceleration, and FIG. 8B shows a hypnogram correlated to FIG. 8A.

Subsequently, as shown in FIG. 9, when generating the learned model IF1, it is found that the accuracy of determination varied depending on the sampling frequency of the teaching data. Specifically, the learned model IF1 is generated with the sampling frequencies of the teaching data as 50 Hz, 25 Hz, 10 Hz and 5 Hz, in the order of FIGS. 8A to 8D.

Overall, there is a tendency for a accuracy score to increase when the sampling frequency of the teaching data is matched with the sampling frequency of the sample signal to be input thereto. In addition, when the sampling frequency of the teaching data used to generate the learned model IF1 is 25 Hz or higher, relatively high accuracy could be achieved even when the sampling frequency of the input sample signal is low. Note that when changing the sampling frequency, no significant difference is observed between when performing resampling processing and when performing thinning processing.

From the above, the learned model IF1 should preferably be a learned model IF1 that is allowed to learn the correlation between the specific frequency component C and the sleep and arousal by acquiring the signal at a sampling frequency of 5 Hz or higher. Further, the sampling frequency of the sample signal input to the learned model IF1 should be 5 Hz or higher. For such a learned model, when the sampling frequency of the sample to be input is matched to that at the time of learning, highly accurate determination can be achieved.

Even more preferably, the learned model IF1 should be a learned model IF1 in which the correlation between the specific frequency component C and the sleep and arousal is learned by acquiring a signal at a sampling frequency of 25 Hz or higher. According to such a manner, a highly accurate determination can be achieved even when the sampling frequency of the sample to be input is lower than that at the time of learning.

That is, the sampling frequency of the signal used for learning of the learned model IF1 is, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 Hz, and may be within a range between any two of the numerical values exemplified here.

Furthermore, as shown in FIG. 10, the bit rate in the analog-to-digital conversion affects accuracy of the determination. According to FIG. 10, in the case of resampling, a difference between 4 bits and 6 bits is observed, and in the case of thinning, a difference between 6 bits and 8 bits is observed. That is, the acquisition unit 331 should preferably acquire the signal by analog-to-digital conversion of the acceleration with a bit rate of 8 bits or more. Specifically, it is, for instance, 8, 10, 12, 14, 16, 32, 48, or 64 bits, and may be within a range between any two of the numerical values illustrated here. According to such a manner, accuracy of the digitally converted signal can be maintained at a high level, thus enabling a more robust determination of sleep and arousal of the user. The analog-to-digital conversion itself may be performed at 8 bits or more, or the acquired signal may be performed in such a manner that the acquired signal is 8 bits or more as a result.

Furthermore, as shown in FIG. 11, the wearable device 2 has been observed to have different frequency sensitivity for each product thereof. Here, the case of a wearable device 2a and a wearable device 2b will be illustrated. In the present embodiment, standardization processing by the correction unit 334 is introduced to mitigate error caused by such variance in frequency sensitivity for each product. By applying the standardization processing, the distribution of frequency sensitivity is changed to an abbreviated uniform distribution for both the wearable device 2a and the wearable device 2b. That is, in the present embodiment, by including the standardization processing by the correction unit 334, influence of frequency sensitivity can be reduced regardless of the product and sleep and arousal of a user can be determined more robustly.

### 4. Others

Regarding the sleep and arousal determination system 1 according to the present embodiment, following aspects may be adopted.

Although the above embodiment is described as a configuration of the sleep and arousal determination system 1, a program may be provided to allow at least one computer to execute each step in the sleep and arousal determination system 1. According to such a manner, it is possible to determine sleep and arousal with sufficiently high accuracy using a small number of wearing device. In addition, since it does not depend on the sampling frequency at which the apparatus acquires signals, sleep and arousal of a user can be determined more robustly.

Log data of the signal indicating acceleration from the wearable device 2 to the information processing apparatus 3 may be exchanged through a communication network such as the Internet, intranet, or dedicated wireless communication, without going through the storage medium M. The measurement of acceleration by the wearable device 2 and the sleep and arousal determination by the information processing apparatus 3 may be performed online in abbreviated real-time with a certain latency.

In the present embodiment, although the acquisition unit 331, the bandwidth limitation unit 332, the conversion unit 333, the correction unit 334, the determination unit 335, and the display controller 336 are described as functional units realized by the controller 33 of the information processing apparatus 3, at least a part thereof may be implemented as a functional unit realized by the controller 23 of the wearable device 2.

Furthermore, the wearable device 2 and the information processing apparatus 3 may be configured as one unit. In other words, the information processing apparatus 3 may be a wearable device 2 that can be worn by a user on a part of body and further comprise an acceleration sensor 24, wherein the acceleration sensor 24 is configured to measure a three-dimensional acceleration vector v (x, y, z) of acceleration.

The bandwidth limitation unit 332 may be configured to limit the frequency component to the specific frequency component C using a band-pass filter instead of the high-pass filter HF. According to such a manner, the specific frequency component can be restricted to a preferred component, and the sleep and arousal of the user can be determined more robustly.

The order of activities A103 and A104 shown in FIG. 5 may be reversed. That is, the correction unit 334 may standardize and correct distribution of the frequency component of the acquired signal, and the bandwidth limitation unit 332 may limit the standardized frequency component to the specific frequency component C. According to such an approach, influence of frequency sensitivity that varies from apparatus to apparatus can be reduced and the sleep and arousal of the user can be determined more robustly.

In addition to the acceleration sensor 24 in the present embodiment, other sensor may be added as appropriate. For instance, SpO2 sensor, ambient light sensor, heart rate sensor, etc. may be added. An SpO2 sensor may be implemented to measure transcutaneous arterial blood oxygen saturation, and a result thereof may be additionally used to determine sleep and arousal. An ambient light sensor may be implemented to measure intensity of ambient light of a user, and a result thereof may be additionally used to determine sleep and arousal. A heart rate sensor may be implemented to measure heart rate of a user, and a result thereof may be additionally used to determine sleep and arousal.

In addition, the present invention may be provided in each of the following aspects.
(1) A sleep and arousal determination system, comprising at least one processor configured to execute a program to cause each of following steps to be performed: an acquisition step of acquiring a signal indicating acceleration of at least a part of body of a user; a bandwidth limitation step of limiting a frequency component included in the signal indicating the acceleration to a specific frequency component; a transform step of Fourier transforming a signal configured of the specific frequency component to generate data for determination; and a determination step of determining sleep and arousal of the user based on the data for determination and preset reference information.
(2) The sleep and arousal determination system according to (1), the processor configured to execute the program to cause following step to be performed: the bandwidth limitation step of limiting to the specific frequency component using a high-pass filter or a band-pass filter.
(3) The sleep and arousal determination system according to (1) or (2), the processor configured to execute the program further to cause following step to be performed: a correction step of standardizing and correcting distribution of frequency component of the data for determination.
(4) The sleep and arousal determination system according to (1) or (2), the processor configured to execute the program further to cause each of following steps to be performed: a correction step of standardizing and correcting distribution of frequency component of the acquired signal, and the bandwidth limitation step of limiting the standardized frequency component to the specific frequency component.
(5) The sleep and arousal determination system according to any one of (1) to (4), the processor configured to execute the program to cause following step to be performed: the acquisition step of acquiring the signal by analog-to-digital conversion of the acceleration with a bit rate of 8 bits or more.
(6) The sleep and arousal determination system according to any one of (1) to (5), wherein: the reference information is a learned model that is allowed to learn correlation between the specific frequency component and the sleep and arousal.
(7) The sleep and arousal determination system according to (6), wherein: the learned model is a learned model in which correlation between the specific frequency component and the sleep and arousal is learned by acquiring the signal at a sampling frequency of 5 Hz or higher.
(8) The sleep and arousal determination system according to (7), wherein: the learned model is a learned model in which correlation between the specific frequency component and the sleep and arousal is learned by acquiring the signal at a sampling frequency of 25 Hz or higher.
(9) The sleep and arousal determination system according to any one of (1) to (8), the processor configured to execute the program to cause following step to be performed: the determination step of specifying a feature quantity for each epoch defined by a predetermined time based on the data for determination, determining sleep and arousal of the user based on, among the epoch, the feature quantity of a desired epoch, the feature quantity of a recent epoch past the desired epoch in time series, and the reference information.
(10) A sleep and arousal determination method, comprising each step in the sleep and arousal determination system according to any one of (1) to (9).
(11) A program that allows at least one computer to execute each step in the sleep and arousal determination system according to any one of (1) to (9).

Of course, the present invention is not limited thereto.

Finally, various embodiments of the present invention have been described, but these are presented as examples and are not intended to limit the scope of the invention. The novel embodiment can be implemented in various other forms, and various omissions, replacements, and changes can be made without departing from the abstract of the invention. The embodiment and its modifications are included in the scope and abstract of the invention and are included in the scope of the invention described in the claims and the equivalent scope thereof.

### REFERENCE SIGNS LIST

1: Sleep and arousal determination system
2: Wearable device
2a: Wearable device
2b: Wearable device
3: Information processing apparatus
20: Communication bus
21: Communication unit
22: Storage unit
23: Controller
24: Acceleration sensor
30: Communication bus
31: Communication unit
32: Storage unit
33: Controller
331: Acquisition unit
332: Bandwidth limitation unit
333: Conversion unit
334: Correction unit
335: Determination unit
336: Display controller
C: Specific frequency component
E: Epoch
HF: High-pass filter
IF: Reference information
IF1: Learned model
M: Storage medium
f: Feature quantity
t: Predetermined time
v: Three-dimensional acceleration vector

## Claims

1. A sleep and arousal determination system, comprising at least one processor configured to execute a program to cause each of following steps to be performed:
an acquisition step of acquiring a signal indicating acceleration of at least a part of body of a user;
a bandwidth limitation step of limiting a frequency component included in the signal indicating the acceleration to a specific frequency component;
a transform step of Fourier transforming a signal configured of the specific frequency component to generate data for determination; and
a determination step of determining sleep and arousal of the user based on the data for determination and preset reference information.

2. The sleep and arousal determination system according to claim 1, the processor configured to execute the program to cause following step to be performed:
the bandwidth limitation step of limiting to the specific frequency component using a high-pass filter or a band-pass filter.

3. The sleep and arousal determination system according to claim 1 or 2, the processor configured to execute the program further to cause following step to be performed:
a correction step of standardizing and correcting distribution of frequency component of the data for determination.

4. The sleep and arousal determination system according to claim 1 or 2, the processor configured to execute the program further to cause each of following steps to be performed:
a correction step of standardizing and correcting distribution of frequency component of the acquired signal, and
the bandwidth limitation step of limiting the standardized frequency component to the specific frequency component.

5. The sleep and arousal determination system according to any one of claims 1 to 4, the processor configured to execute the program to cause following step to be performed:
the acquisition step of acquiring the signal by analog-to-digital conversion of the acceleration with a bit rate of 8 bits or more.

6. The sleep and arousal determination system according to any one of claims 1 to 5, wherein:
the reference information is a learned model that is allowed to learn correlation between the specific frequency component and the sleep and arousal.

7. The sleep and arousal determination system according to claim 6, wherein:
the learned model is a learned model in which correlation between the specific frequency component and the sleep and arousal is learned by acquiring the signal at a sampling frequency of 5 Hz or higher.

8. The sleep and arousal determination system according to claim 7, wherein:
the learned model is a learned model in which correlation between the specific frequency component and the sleep and arousal is learned by acquiring the signal at a sampling frequency of 25 Hz or higher.

9. The sleep and arousal determination system according to any one of claims 1 to 8, the processor configured to execute the program to cause following step to be performed:
the determination step of
specifying a feature quantity for each epoch defined by a predetermined time based on the data for determination,
determining sleep and arousal of the user based on, among the epoch, the feature quantity of a desired epoch, the feature quantity of a recent epoch past the desired epoch in time series, and the reference information.

10. A sleep and arousal determination method, comprising each step in the sleep and arousal determination system according to any one of claims 1 to 9.

11. A program that allows at least one computer to execute each step in the sleep and arousal determination system according to any one of claims 1 to 9.
